# EUROPÄISCHE PATENTSCHRIFT

(11) **EP 2 059 280 B1**
(45) Veröffentlichungstag und Bekanntmachung des Hinweises auf die Patenterteilung: **27.10.2010**
(21) Anmeldenummer: 07818056.9
(22) Anmeldetag: 06.09.2007
(51) Int. Cl.: A61M 1/36

(54) **Vorrichtung zur Überwachung eines Zugangs zu einem Patienten, insbesondere eines Gefässzugangs bei einer extrakorporalen Blutbehandlung**
Device for monitoring an access to a patient, in particular a vascular access in extracorporeal blood treatment
Dispositif de surveillance d'un accès à un patient, en particulier, d'un accès vasculaire dans un traitement sanguin extracorporel

(30) Priorität: 08.09.2006 DE 102006042336
(43) Veröffentlichungstag der Anmeldung: 20.05.2009
(73) Patentinhaber: Fresenius Medical Care Deutschland GmbH, 61352 Bad Homburg v.d.H. (DE)
(72) Erfinder: KÖNIG, Christoph, 65207 Wiesbaden/Auringen (DE); KLEINEKOFORT, Wolfgang, 65779 Kelkheim (DE)
(74) Vertreter: Luderschmidt, Schüler & Partner
(86) Internationale Anmeldenummer: PCT/EP2007/007763
(87) Internationale Veröffentlichungsnummer: WO 2008/028653

(56) Entgegenhaltungen:
- WO-A-01/47581
- WO-A-02/102441
- DE-C1- 19 739 099
- US-A1- 2003 195 454

## Beschreibung

Die Erfindung betrifft eine Vorrichtung zur Überwachung eines Zugangs zu einem Patienten, wobei über eine erste Schlauchleitung, die einen ersten Patientenanschluss aufweist, eine Flüssigkeit dem Patienten entnommen und über eine zweite Schlauchleitung, die einen zweiten Paüentenanschluss aufweist, die Flüssigkeit dem Patienten wieder zugeführt wird, insbesondere zur Überwachung eines Gefäßzugangs bei einer extrakorporalen Blutbehandlung, bei der Blut eines Patienten über eine arterielle Schlauchleitung, die einen arteriellen Patientenanschluss mit einer arteriellen Punktionskanüle aufweist, dem Patienten entnommen und über eine venöse Schlauchleitung, die einen venösen Patientenanschluss mit einer venösen Punktionskanüle aufweist, dem Patienten wieder zugeführt wird.

Auf dem Gebiet der Medizintechnik sind eine Vielzahl von Einrichtungen bekannt, mit denen über eine Schlauchleitung einem Patienten Flüssigkeiten entnommen oder Flüssigkeiten dem Patienten zugeführt werden können. Dabei erfolgt der Zugang zu dem Patienten im Allgemeinen mit einem Katheter zum Einführen in Körperorgane oder eine Kanüle zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Bei Methoden der Blutreinigungstherapie wie Hämodialyse, Hämofiltration und Hämodiafiltration wird Blut über einen extrakorporalen Blutkreislauf geleitet. Falls sich der venöse Patientenanschluss während der Blutbehandlung löst, kann ein Verbluten des Patienten nur verhindert werden, wenn innerhalb weniger Sekunden der extrakorporale Blutfluss gestoppt wird. Daher sind extrakorporale Blutkreisläufe in der Regel mit Schutzsystemen ausgestattet, die im Alarmfall die Blutpumpe stoppen, die venöse Klemme schließen sowie ein akustisches oder optisches Warnsignal auslösen.

Die DE 197 39 099 C1 beschreibt eine Vorrichtung zur Überwachung eines Zugangs während einer extrakorporalen Blutbehandlung, bei der ein elektrischer Strom in der eine geschlossene Leiterschleife darstellenden Verbindung des extrakorporalen Blutkreislaufs induziert wird, der in der Leiterschleife fließende Strom gemessen und bei einer charakteristischen Veränderung der Stromstärke auf einen fehlerhaften Gefäßzugang geschlossen wird. Neben der induktiven Ein- und Auskopplung ist es auch bekannt, elektrische Signale in den extrakorporalen Blutkreislauf kapazitiv ein- und auszukoppeln.

Die US 6,932,786 B2 beschreibt eine Überwachungsvorrichtung, bei der ein Wechselspannungssignal in den extrakorporalen Blutkreislauf kapazitiv ein- und ausgekoppelt wird. Die Ein- und Auskopplung des Wechselspannungssignals erfolgt mittels die Schlauchleitungen umschließender elektrischer Kontaktelemente. Dabei stellt das elektrische Kontaktelement die eine "Elektrode" eines "Kondensators" dar, während das in den Schlauchleitungen strömende Blut die andere "Elektrode" des "Kondensators" bildet. Die isolierende Schlauchleitung stellt das zwischen den Elektroden liegende Dielektrikum des Kondensators dar.

Bei der bekannten Überwachungsvorrichtung wird das von einem Wechselspannungsgenerator erzeugte Wechselspannungssignal mit einem venösen Kontaktelement an der venösen Blutleitung und einem arteriellen Kontaktelement an der arteriellen Blutleitung als Differenzsignal eingekoppelt. Eine alternative Ausführungsform sieht vor, dass der eine Ausgang des Frequenzgenerators mit einem die venöse Blutleitung umschließenden Kontaktelement verbunden ist, während der andere Ausgang des Signalgenerators auf Massepotential liegt. Beide Ausführungsformen beruhen darauf, dass die Auskopplung des Wechselspannungssignals als Differenzsignal mit zwei Kontaktelementen erfolgt, die an unterschiedlichen Stellen des extrakorporalen Kreislaufs angeordnet sind, wobei das im extrakorporalen Kreislauf strömende Blut auf Massepotential liegt.

Es hat sich bei Versuchen herausgestellt, dass bei dem aus der US 6,932,786 B2 bekannten Verfahren das ausgekoppelte Wechselspannungssignal von relativ starken Störsignalen überlagert werden kann. In der Praxis kann sich die bekannte Vorrichtung daher als relativ störanfällig erweisen.

Die US 2003/0195454 A1 setzt sich mit der Problematik der kapazitiven Ein- und Auskopplung von Messsignalen in den extrakorporalen Blutkreislauf auseinander und schlägt vor, die Messsignale mittels elektrischer Kontaktelemente, die mit dem durch die Schlauchleitungen strömenden Blut unmittelbar in Kontakt stehen, in den extrakorporalen Kreislauf ein- und auszukoppeln.

Die US 7,060,047 beschreibt eine Vorrichtung zur Überwachung eines Gefäßzugangs bei einer Dialysebehandlung, die eine kapazitive Einkopplung eines Wechselspannungssignals vorsieht, wobei ein Stromkreis über einen gemeinsamen Erdschluss geschlossen wird. Die Vorrichtung sieht dabei grundsätzlich eine Verbindung des Patienten mit der Erde vor. Allerdings wird in der Druckschrift darauf hingewiesen, dass eine derartige Ankopplung des Patienten nicht zwingend erforderlich ist.

Der Erfindung liegt die Aufgabe zu Grunde, eine Vorrichtung zu schaffen, die eine Überwachung des Zugangs zu einem Patienten mit hoher Sicherheit erlaubt, obwohl die Ein- und Auskopplung des Messsignals kapazitiv erfolgt. Darüber hinaus ist eine Aufgabe der Erfindung, eine Blutbehandlungsvorrichtung mit einer Vorrichtung zur Überwachung eines Patientenzugangs bereit zu stellen, die eine Überwachung des Patientenzugangs mit hoher Sicherheit ermöglicht.

Die Lösung dieser Aufgaben erfolgt mit den Merkmalen der Patentansprüche 1 und 5. Vorteilhafte Ausführungsformen der Erfindung sind Gegenstand der Unteransprüche.

Die erfindungsgemäße Überwachungsvorrichtung unterscheidet sich von den aus dem Stand der Technik bekannten Überwachungsvorrichtungen dadurch, dass das Wechselspannungssignal bezogen auf ein gemeinsames Massepotential night nur sondem auch ausgekoppelt wird. Zudem findet eine Differenzmessung nicht statt. Es hat sich überraschend herausgestellt, dass bei einer Ein- und Auskopplung des Wechselspannungssignals in Bezug auf ein gemeinsames Massepotential die Störsignale verringert werden, die ansonsten insbesondere bei den unvermeidbaren Schlauchbewegungen auftreten könnten.

Bei der erfindungsgemäßen Vorrichtung wird das Wechselspannungssignal nur an einer Stelle einer der beiden Schlauchleitungen eingekoppelt und nur an einer Stelle der anderen der beiden Schlauchleitungen ausgekoppelt. Damit reduziert sich auch der Aufwand für die kapazitive Ein- und Auskopplung des Spannungssignals.

Die Mittel zur kapazitiven Ein- und Auskopplung des Wechselspannungssignals sind vorzugsweise die Schlauchleitungen umschließende Körper aus elektrische leitendem Material, beispielsweise Hülsen aus Metall.

Ein nicht ordnungsgemäßer Patientenzugang, beispielsweise das Herausrutschen der venösen oder arteriellen Punktionskanüle aus der venösen bzw. arteriellen Blutleitung, hat eine Änderung der Impedanz zur Folge, die wiederum zu einer Änderung der Amplitude des ausgekoppelten Wechselspannungssignals führt. Folglich kann bei einer charakteristischen Änderung der Amplitude des gemessenen Wechselspannungssignals, vorzugsweise einer Verringerung dessen Amplitude, auf einen nicht ordnungsgemäßen Gefäßzugang geschlossen werden.

Bei einer extrakorporalen Blutbehandlung kann ein nicht ordnungsgemäßer Gefäßzugang nicht nur dann vorliegen, wenn die arterielle und/oder venöse Punktionskanüle aus der arteriellen bzw. venösen Blutleitung herausgerutscht ist, sondern auch dann, wenn die Blutleitung unterbrochen ist. Die bekannten arteriellen und venösen Blutleitungen verfügen im Allgemeinen über Schlauchverbindungen, die zwei Schlauchabschnitte stromauf bzw. stromab der venösen bzw. arteriellen Punktionskanüle miteinander verbinden. Diese Schlauchverbindungen sind im Allgemeinen Luer-Lock-Verbindungen. Wenn sich die Schlauchverbindung lösen sollte, ist ein Zugang zu dem Gefäß nicht mehr gegeben. Auch dies kann mit der erfindungsgemäßen Vorrichtung nachgewiesen werden. Die erfindungsgemäße Vorrichtung sieht das Ein- bzw. Auskoppeln des Wechselspannungssignals gegenüber dem Massepotential an einer beliebigen Stelle der arteriellen bzw. venösen Blutleitung, d.h. entweder stromauf oder stromab der Schlauchverbindung, beispielsweise Luer-Lock-Verbindung, d.h. in dem Schlauchabschnitt zwischen dem Eintritt in den Dialysator bzw. Austritt aus dem Dialysator und der Schlauchverbindung oder dem Schlauchabschnitt zwischen Schlauchverbindung und Punktionskanüle vor. Wenn die Ein- bzw. Auskopplung des Wechselspannungssignals in den Schlauchleitungsabschnitten zwischen Schlauchverbindung und Punktionskanüle erfolgt, kann nur ein Herausrutschen der Punktionskanüle, nicht aber eine fehlerhafte Schlauchverbindung nachgewiesen werden. Der Nachweis einer fehlerhaften Schlauchverbindung setzt voraus, dass eine Einkopplung oder Auskopplung in einem Schlauchleitungsabschnitt zwischen Dialysator und Schlauchverbindung erfolgt.

Im Folgenden werden Ausführungsbeispiele der Erfindung unter Bezugnahme auf die Zeichnungen näher erläutert.

Es zeigen:
- Figur 1: die wesentlichen Komponenten einer Blutbehandlungsvorrichtung zusammen mit einer Vorrichtung zur Überwachung des Patientenzugangs in stark vereinfachter schematischer Darstellung,
- Figur 2: das Ersatzschaltbild der Blutbehandlungsvorrichtung von Figur 1,
- Figur 3: das Ersatzschaltbild einer alternativen Ausführungsform der Blutbehandlungsvorrichtung, bei der die Einkopplung des Wechselspannungssignals stromab einer arteriellen Schlauchverbindung und die Auskopplung des Wechselspannungssignals stromauf einer venösen Schlauchverbindung erfolgt, wobei die arterielle und venöse Schlauchverbindung jeweils zwei Schlauchabschnitte der arteriellen oder venösen Schlauchleitung verbindet,
- Figur 4: eine weitere Ausführungsform der Blutbehandlungsvorrichtung, bei der die Einkopplung des Wechselspannungssignals stromab der arteriellen Schlauchverbindung und die Auskopplung des Wechselspannungssignals stromab der venösen Schlauchverbindung erfolgt und
- Figur 5: eine weitere Ausführungsform der Blutbehandlungsvorrichtung, bei der die Einkopplung des Wechselspannungssignals stromauf der arteriellen Schlauchverbindung und die Auskopplung des Wechselspannungssignals stromauf der venösen Schlauchverbindung erfolgt.

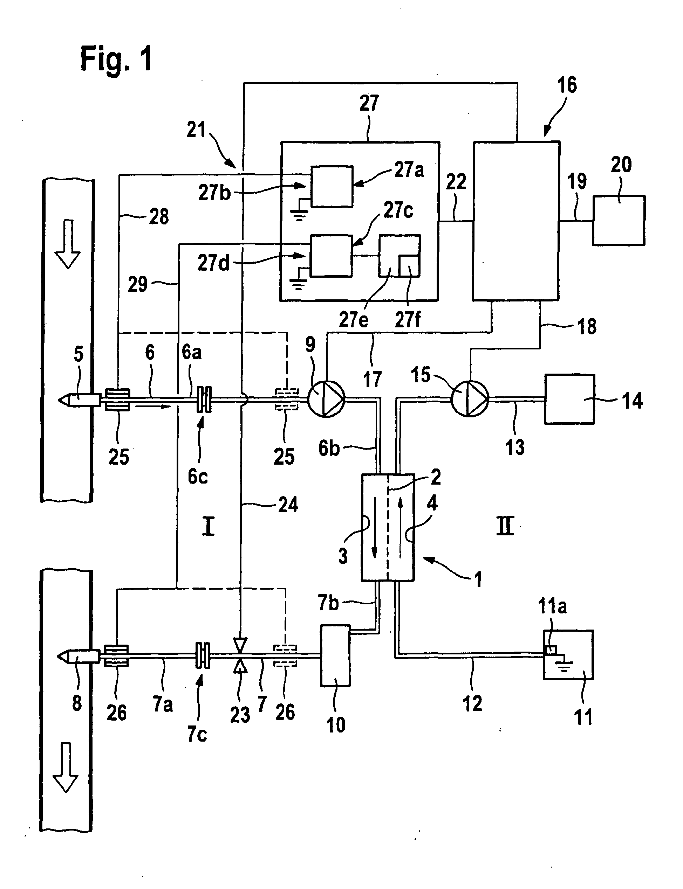

Figur 1 zeigt die wesentlichen Komponenten einer Blutbehandlungsvorrichtung, beispielsweise einer Hämodialysevorrichtung, die über eine Vorrichtung zur Überwachung des arteriellen und venösen Gefäßzugangs verfügt. Die Hämodialysevorrichtung weist einen Dialysator 1 auf, der durch eine semipereable Membran 2 in eine Blutkammer 3 und eine Dialysierflüssigkeitskammer 4 unterteilt ist. An einer Arterie des Patienten ist mittels einer arteriellen Punktionskanüle 5 eine arterielle Schlauchleitung 6 angeschlossen, die zu dem Einlass der Blutkammer 3 des Dialysators 1 führt. Von dem Auslass der Blutkammer 3 des Dialysators 1 geht eine venöse Schlauchleitung 7 ab, die mittels einer venösen Punktionskanüle 8 an einer Vene des Patienten angeschlossen ist. Die arterielle Schlauchleitung 6 ist in eine okkludierende Blutpumpe 9 eingelegt, die das Blut im extrakorporalen Blutkreislauf I fördert. In der venösen Schlauchleitung 7 befindet sich ein Blasenfänger 10, beispielsweise eine Tropfkammer, die Luftblasen im Blut zurückhält.

Der Dialysierflüssigkeitskreislauf II der Hämodialysevorrichtung umfaßt eine Einrichtung 11 zur Bereitstellung der Dialysierflüssigkeit, an der eine Dialysierflüssigkeitszuführleitung 12 angeschlossen ist, die zu dem. Einlass der Dialysierflüssigkeitskammer 4 des Dialysators 1 führt. Von dem Auslass der Dialysierflüssigkeitskammer 4 des Dialysators 1 geht eine Dialysierflüssigkeitsabführleitung 13 ab, die zu einem Auslass 14 führt. In die Dialysierflüssigkeitsabführleitung 13 ist eine Dialysierflüssigkeitspumpe 15 geschaltet.

Die arterielle und venöse Schlauchleitung 6, 7 sind Teil eines Schlauchsets, wobei die arterielle und venöse Schlauchleitung jeweils zwei Schlauchleitungsabschnitte 6a, 6b und 7a, 7b umfassen. Die Leitungsabschnitte 6a, 6b der arteriellen Schlauchleitung 6 und die Leitungsabschnitte 7a, 7b der venösen Schlauchleitung 7 sind mit Schlauchverbindungen 6c, 7c, beispielsweise Luer-Lock-Verbindungen, miteinander verbunden, so dass die patientenseitigen Schlauchabschnitte von den übrigen Leitungsabschnitten des Schlauchsets getrennt werden können.

Die Steuerung der Dialysevorrichtung übernimmt eine zentrale Steuereinheit 16, die über Steuerleitungen 17, 18 die Blut- und Dialysierflüssigkeitspumpe 9, 15 ansteuert. Die zentrale Steuereinheit 16 ist über eine Datenleitung 19 mit einer Alarmeinheit 20 verbunden, die bei einem Störfall einen optischen und/oder akustischen Alarm gibt.

Ein ordnungsgemäßer Gefäßzugang setzt voraus, dass sich sowohl die arterielle als auch venöse Punktionskanüle 5, 8 im Gefäß befinden. Weiterhin setzt ein ordnungsgemäßer Gefäßzugang voraus, dass die Schlauchverbindungen 6c, 7c der arteriellen und venösen Schlauchleitung 6, 7 die beiden Schlauchleitungsabschnitte miteinander verbinden.
Zur Überwachung des Gefäßzugangs weist die Dialysevorrichtung eine Überwachungseinrichtung 21 auf, die über eine Datenleitung 22 mit der Steuereinheit 16 kommuniziert. Der Aufbau und die Funktionsweise der Überwachungseinrichtung 21 werden nachfolgend noch im Einzelnen beschrieben. Die Feststellung eines nicht ordnungsgemäßen Gefäßzugangs signalisiert die Überwachungseinrichtung 21 der Steuereinheit 16 über eine Datenleitung 22, so dass die Steuereinheit 16 die Alarmeinheit 20 ansteuert, die einen optischen und/oder akustischen Alarm gibt. Weiterhin schließt die Steuereinheit 16 eine an der venösen Schlauchleitung 7 stromab der Blutkammer 3 des Dialysators 1 angeordnete venösen Schlauchklemme 23, die über eine Steuerleitung 24 mit der Steuereinheit 16 verbunden ist.

Die Überwachungseinrichtung 21 weist Mittel 25 zur kapazitiven Einkopplung eines Wechselspannungssignals und Mittel 26 zur kapazitiven Auskopplung eines Wechselspannungssignals sowie eine Rechen- und Auswerteinheit 27 auf. Bei den Mitteln zum kapazitiven Ein- und Auskoppeln des Wechselspannungssignals handelt es sich um die Schlauchleitungen umschließende Metallhülsen.

Bei dem Ausführungsbeispiel gemäß Figur 1 umschließt die arterielle Metallhülse 25 den arteriellen Schlauchleitungsabschnitt zwischen arterieller Punktionskanüle 5 und arterieller Schlauchverbindung 6c, während die venöse Metallhülse 26 den venösen Schlauchleitungsabschnitt zwischen venöser Schlauchverbindung 7c und venöser Punktionskanüle 8 umschließt. Es ist aber auch möglich, die arterielle Metallhülse 25 in dem arteriellen Schlauchleitungsabschnitt zwischen der arteriellen Schlauchverbindung 6c und der Blutkammer 3 vorzugsweise stromauf der Blutpumpe 9 und die venöse Metallhülse 26 in dem venösen Schlauchleitungsabschnitt zwischen der Blutkammer 3 und der venösen Schlauchverbindung 7c vorzugsweise stromauf der Schlauchklemme 23 anzuordnen. Diese Anordnung ist in Figur 1 in gestrichelten Linien dargestellt.
Die Einrichtung 11 zur Bereitstellung der Dialysierflüssigkeit sieht vor, dass die Dialysierflüssigkeit auf Massepotential, d.h. auf die Betriebserde der Maschine gelegt wird. Hierzu ist in der Einrichtung 11 zur Bereitstellung der Dialysierflüssigkeit ein nur andeutungsweise dargestelltes elektrisches Kontaktelement 11a, beispielsweise eine Erdungschelle vorgesehen, die mit der Dialysierflüssigkeit in Berührung steht. Da die Dialysierflüssigkeit wiederum über die Membran 2 des Dialysators 1 mit dem Blut in Verbindung steht, wird mit der Erdung der Dialysierflüssigkeit auch das durch die Schlauchleitungen 6, 7 strömende Blut auf Massepotential gelegt, d.h. mit der Betriebserde der Maschine verbunden ist.

Die Überwachungsvorrichtung 21 weist Mittel 27a zum Erzeugen eines Wechselspannungssignals mit einem Signalausgang 27b auf, wobei der eine Anschluss des Signalausgangs über eine elektrische Verbindungsleitung 28 mit der arteriellen Metallhülse 25 verbunden ist, während der andere Anschluss des Signalausgangs auf Massepotential liegt, d.h. mit der Betriebserde der Dialysemaschine verbunden ist. Darüber hinaus weist die Überwachungseinrichtung Mittel 27c zum Messen eines Wechselspannungssignals mit einem Signaleingang 27d auf. Der eine Anschluss des Signaleingangs 27d ist über eine elektrische Verbindungsleitung 29 mit der venösen Metallhülse 26 verbunden, während der andere Anschluss des Signaleingangs wieder auf Massepotential liegt, d.h. mit der Betriebserde der Maschine verbunden.

Des weiteren verfügt die Überwachungsvorrichtung 21 über Mittel 27e zum Auswerten des mit dem Mitteln 27c gemessenen Wechselspannungssignals. Die Mittel 27e zum Auswerten des Wechselspannungssignals wiederum weisen Mittel 27f zum Vergleichen des gemessenen Wechselspannungssignals mit einem vorgegebenen Grenzwert auf.

Die erfindungsgemäße Überwachungsvorrichtung arbeitet wie folgt. Es wird ein Wechselspannungssignal erzeugt, das an der arteriellen Schlauchleitung 6 kapazitiv in den extrakorporalen Blutkreislauf I eingekoppelt und an der venösen Schlauchleitung 7 kapazitiv aus dem extrakorporalen Blutkreislauf wieder ausgekoppelt wird. Der Messstrom, der beim Anliegen des Wechselspannungssignals über den Blutschlauch in den Patienten fließt, ist vernachlässigbar. Die Amplitude des ausgekoppelten Wechselspannungssignals wird mit einem vorgegebenen Grenzwert verglichen. Wenn die Amplitude des Spannungssignals kleiner als der vorgegebene Grenzwert ist, schließt die Überwachungsvorrichtung 21 auf einen nicht ordnungsgemäßen Gefäßzugang, so dass Alarm gegeben und der extrakorporale Blutkreislauf unterbrochen wird.

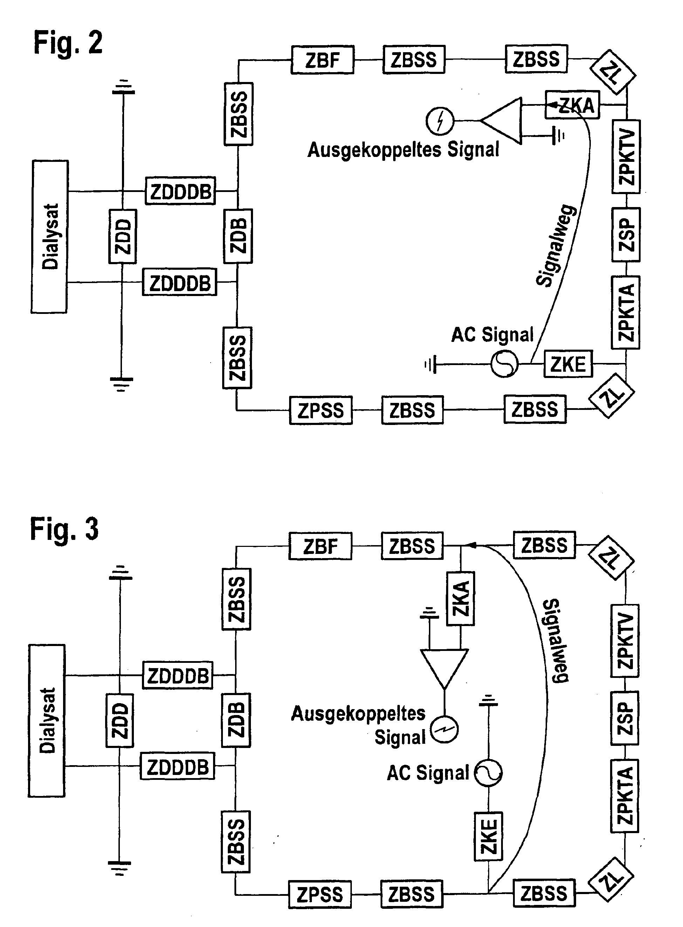

Die Figuren 2 bis 4 zeigen die elektrischen Ersatzschaltbilder der Dialysevorrichtung von Figur 1 für unterschiedliche Anordnungen der Mittel zum Ein- bzw. Auskoppeln des Wechselspannungssignals.

In den Figuren 2 bis 4 werden die einzelnen Komponenten der Dialysevorrichtung durch ihre Impedanz Z beschrieben, die mit diskreten Komponenten als Serienschaltung eines Widerstandes Rb und einer Parallelschaltung eines Widerstandes Ra und eines Kondensators Cx dargestellt werden kann. Für die Impedanzen der einzelnen Komponenten finden die folgenden Abkürzungen Verwendung:
- ZDD =: Impedanz Dialysator Dialysatseite
- ZDB =: Impedanz Dialysator Blutseite
- ZDDDB =: Impedanz Dialysatseite → Blutseite
- ZBSS =: Impedanz Blutschlauchsegment
- ZBF =: Impedanz Blasenfänger
- ZKA =: Impedanz kapazitive Auskopplung
- ZL =: Impedanz Schauchverbindung (Luer-Lock)
- ZKE =: Impedanz kapazitive Einkopplung
- ZPSS =: Impedanz Pumpschlauchsegment
- ZSP =: Impedanz Shunt Patient
- ZPKTV =: Impedanz Punktion venös
- ZPKTA =: Impedanz Punktion arteriell

Das Lösen der arteriellen oder venösen Punktionskanüle 5, 8 bedeutet eine Unterbrechung des "Stromkreises". Auch ein Lösen der arteriellen oder venösen Schlauchverbindung bedeutet eine Unterbrechung des Stromkreises. Die Unterbrechung des Stromkreises hat eine Erhöhung der Impedanz zufolge, die wiederum durch eine Verringerung der Amplitude des gemessenen Wechselspannungssignals nachgewiesen wird. Der Signalweg zwischen der Ein- und Auskoppelstelle ist in den Figuren 2 bis 5 durch eine geschwungene Linie angedeutet.

Figur 2 zeigt die in Fig. 1 mit durchgezogenen Linien dargestellte Anordnung der Metallhülsen 25, 26 zum Ein- und Auskoppeln des Wechselspannungssignals. Mit dieser Anordnung kann nur eine Diskonnektion oder wenigstens Dislokation der arteriellen und venösen Kanüle 5, 8 aufgrund einer signifikanten Erhöhung der Impedanz bzw. Verringerung der Amplitude des Wechselspannungssignals mit hoher Sicherheit erkannt werden, nicht aber das Lösen der Schlauchverbindungen nachgewiesen werden.

Figur 3 zeigt die in Fig. 1 mit gestrichelten Linien dargestellte Anordnung der arteriellen und venösen Metallhülse 25, 26. Mit dieser Anordnung kann nur eine Diskonnektion oder wenigstens Dislokation der arteriellen und venösen Kanüle 5, 8 aufgrund einer signifikanten Erhöhung der Impedanz bzw. Verringerung der Amplitude des Wechselspannungssignals mit hoher Sicherheit erkannt werden, nicht aber das Lösen der Schlauchverbindungen mit hoher Sicherheit nachgewiesen werden. Das Lösen einer Schlauchverbindung führt hingegen nur zu einem kleinen Signalanstieg, da die Ableitung gegen Masse über die Impedanz ZDDDB fehlt.

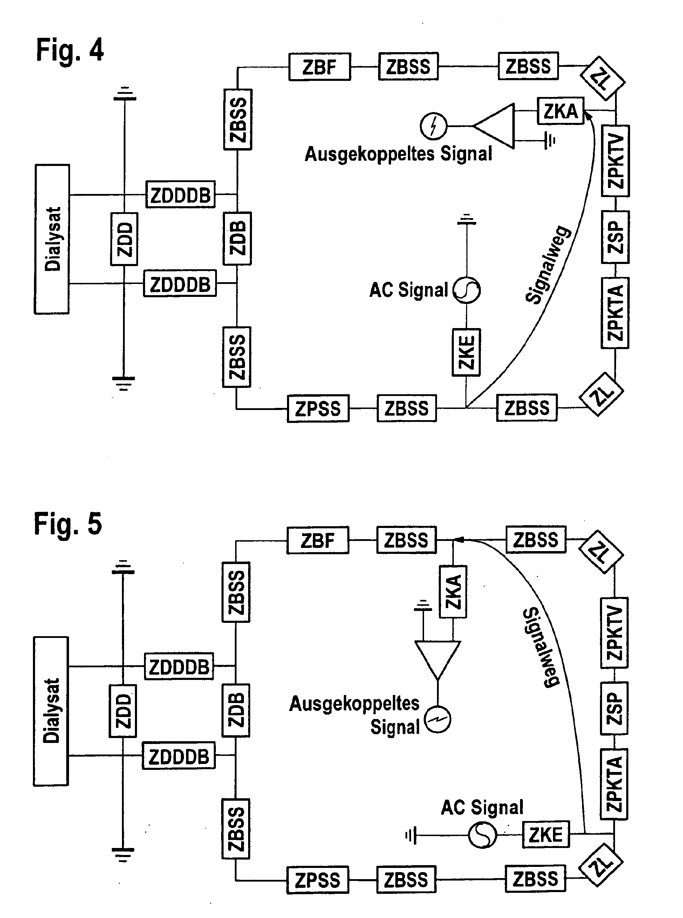

Figur 4 zeigt ein Ausführungsbeispiel, bei dem die arterielle Metallhülse 25 in dem Schlauchleitungsabschnitt 6a der arteriellen Schlauchleitung 6 zwischen der Blutkammer 3 und der arteriellen Schlauchverbindung 6c angeordnet ist, während die venöse Metallhülse 26 in dem Schlauchleitungsabschnitt 7a der venösen Schlauchleitung 7 zwischen der venösen Punktionskanüle 8 und der venösen Schlauchverbindung 7c angeordnet ist (Fig. 1).

Figur 5 zeigt die Anordnung der arteriellen Metallhülse 25 in dem arteriellen Schlauchleitungsabschnitt 6a zwischen der arteriellen Punktionskanüle 5 und der arteriellen Schlauchverbindung 6c, während die venöse Metallhülse 26 in dem venösen Schlauchleitungsabschnitt 7a zwischen der Blutkammer 3 und der venösen Schlauchverbindung 7c angeordnet ist.

Bei den Ausführungsbeispielen gemäss der Figuren 4 und 5 wird das Lösen sowohl der Punktionskanüle 5, 8 als auch der Schlauchverbindung 6c, 7c durch eine Erhöhung der Impedanz und einer Verringerung der Amplitude des gemessenen Wechselspannungssignals nachgewiesen.

Bei der erfindungsgemäßen Überwachungsvorrichtung kann ein Vergleich der Amplitude des gemessenen Wechselspannungssignals nicht nur mit einem vorgegebenen Referenzwert, sondern mit mehreren Referenzwerten vorgesehen sein. Damit ist grundsätzlich bei entsprechender Anordnung der Ein- und Auskoppelstellen eine Unterscheidung möglich, ob sich eine Punktionskanüle oder eine Schlauchverbindung gelöst hat, da der jeweilige Störfall mit einer charakteristischen Änderung der Impedanz oder der Signalamplitude verbunden ist. Die Größe der signifikanten Änderung ergibt sich aus dem jeweiligen Ersatzschaltbild. Charakteristische Werte können durch Vergleichsmessungen festgelegt werden.

## Patentansprüche

1. Vorrichtung zur Überwachung eines Zugangs zu einem Patienten, wobei über eine erste Schlauchleitung, die einen ersten Patientenanschluss aufweist, eine Flüssigkeit dem Patienten entnommen und über eine zweite Schlauchleitung, die einen zweiten Patientenanschluss aufweist, die Flüssigkeit dem Patienten wieder zugeführt wird, insbesondere zur Überwachung eines Gefäßzugang bei einer extrakorporalen Blutbehandlung, bei der Blut eines Patienten über eine arterielle Schlauchleitung, die einen arteriellen Patientenanschluss mit einer arteriellen Punktionskanüle aufweist, dem Patienten entnommen und über eine venöse Schlauchleitung, die einen venösen Patientenanschluss mit einer venösen Punktionskanüle aufweist, dem Patienten wieder zugeführt wird,
wobei die Überwachungsvorrichtung aufweist:
Mittel (27a) zum Erzeugen eines Wechselspannungssignals, die einen Signalausgang (27b) mit einem ersten und einem zweiten Anschluss aufweisen,
Mittel (25) zur kapazitiven Einkopplung des Wechselspannungssignals an einer ersten Stelle einer der beiden Schlauchleitungen,
Mittel (26) zur kapazitiven Auskopplung des Wechselspannungssignals an einer zweiten Stelle der anderen der beiden Schlauchleitungen,
Mittel (27c) zum Messen eines Wechselspannungssignals, die einen Signaleingang (27d) mit einem ersten und einem zweiten Anschluss aufweisen, wobei der erste Anschluss des Signaleingangs (27d) der Mittel (27c) zum Messen eines Wechselspannungssignals mit den Mitteln (26) zur kapazitiven Auskopplung des Wechselspannungssignals elektrisch verbunden ist;
Mittel (28) zur Herstellung einer elektrischen Verbindung zwischen dem ersten Anschluss des Signalausgangs (27b) der Mittel (27a) zum Erzeugen eines Wechselspannungssignals und den Mitteln (25) zur kapazitiven Einkopplung des Wechselspannungssignals und zur Herstellung einer elektrischen Verbindung zwischen dem zweiten Anschluss des Signalausgangs (27b) der Mittel (27a) zum Erzeugen eines Wechselspannungssignals und Masse;
Mittel (II, 11, 11a) zur Herstellung einer elektrischen Verbindung zwischen der Flüssigkeit in den Schlauchleitungen und Masse und
Mittel (27e) zum Auswerten des gemessenen Wechselspannungssignals, die derart mit den Mitteln (27c) zum Messen eines Wechselspannungssignals zusammenwirken, dass auf einen nicht ordnungsgemäßen Gefäßzugang bei einer charakteristischen Änderung der Amplitude des gemessenen Wechselspannungssignals geschlossen wird;
**dadurch gekennzeichnet, dass**
der zweite Anschluss des Signaleingangs (27d) der Mittel zum Messen (27c) eines Wechselspannungssignals mit Masse elektrisch verbunden ist.

2. Vorrichtung nach Anspruch 1, **dadurch gekennzeichnet, dass** die Mittel (25) zur kapazitiven Einkopplung des Wechselspannungssignals einen ersten Körper aus elektrisch leitendem Material zum Umschließen einer der beiden Schlauchleitungen (6) an einer ersten Stelle aufweisen.

3. Vorrichtung nach Anspruch 1 oder 2, **dadurch gekennzeichnet, dass** die Mittel (26) zur kapazitiven Auskopplung des Wechselspannungssignals einen zweiten Körper aus elektrisch leitendem Material zum Umschließen der anderen der beiden Schlauchleitungen (7) an einer zweiten Stelle aufweisen.

4. Vorrichtung nach einem der Ansprüche 1 bis 3, **dadurch gekennzeichnet, dass** die Mittel (27e) zum Auswerten des gemessenen Wechselspannungssignals Mittel (27f) zum Vergleichen des gemessenen Wechselspannungssignals mit einem vorgegebenen Grenzwert aufweisen, wobei auf einen nicht ordnungsgemäßen Gefäßzugang geschlossen wird, wenn das Wechselspaunungssignal kleiner als der Grenzwert ist.

5. Blutbehandlungsvorrichtung mit einem Dialysierflüssigkeitskreislauf (II), der eine Dialysierflüssigkeitskammer (4) eines von einer semipermeablen Membran (2) in eine Blutkammer (3) und die Dialysierflüssigkeitskammer (4) unterteilten Dialysators (1) einschließt und einen Blutkreislauf (I), der die Blutkammer (3) des Dialysators (1) einschließt, wobei eine arterielle Schlauchleitung (6), die an einer arteriellen Punktionskanüle (5) angeschlossen ist, zu der Blutkammer des Dialysators führt und eine venöse Schlauchleitung (7), die an einer venösen Punktionskanüle (8) angeschlossen ist, von der Blutkammer des Dialysators abgeht, und mit einer Vorrichtung zur Überwachung des Gefäßzugangs nach einem der Ansprüche 1 bis 4.

6. Blutbehandlungsvorrichtung nach Anspruch 5, **dadurch gekennzeichnet, dass** die Mittel zur Herstellung einer elektrischen Verbindung zwischen der Flüssigkeit in den Schlauchleitungen und Masse im Dialysierflüssigkeitskreislauf (I) angeordnet sind.

7. Blutbehandlungsvorrichtung nach Anspruch 6, **dadurch gekennzeichnet, dass** die Mittel zur Herstellung einer elektrischen Verbindung zwischen der Flüssigkeit in den Schlauchleitungen und Masse ein mit der Dialysierflüssigkeit in Berührung stehendes elektrisches Kontaktelement (11a) sind, das auf Massepotential gelegt ist.

8. Blutbehandlungsvorrichtung nach einem der Ansprüche 5 bis 8, **dadurch gekennzeichnet, dass** die Mittel (25) zur kapazitiven Einkopplung des Wechselspannungssignals stromab der arteriellen Punktionskanüle (5) an einer ersten Stelle der arteriellen Blutleitung (6) angeordnet sind.

9. Blutbehandlungsvorrichtung nach einem der Ansprüche 5 bis 8, **dadurch gekennzeichnet, dass** die arterielle Blutleitung (6) stromab der arteriellen Punktionskanüle (5) eine Schlauchverbindung (6c) zwischen einem ersten und einem zweiten Abschnitt aufweist, wobei die Mittel zur kapazitiven Einkopplung (25) des Wechselspannungssignals in dem ersten Abschnitt zwischen Punktionskanüle (5) und Schlauchverbindung (6c) angeordnet sind.

10. Blutbehandlungsvorrichtung nach einem der Ansprüche 5 bis 8, **dadurch gekennzeichnet, dass** die arterielle Blutleitung (6) stromab der arteriellen Punktionskanüle (5) eine Schlauchverbindung (6c) zwischen einem ersten und einem zweiten Abschnitt aufweist, wobei die Mittel (25) zur kapazitiven Einkopplung des Wechselspannungssignals in dem zweiten Abschnitt zwischen Schlauchverbindung (6c) und Blutkammer (3) angeordnet sind.

11. Blutbehandlungsvorrichtung nach einem der Ansprüche 5 bis 8, **dadurch gekennzeichnet, dass** die Mittel (26) zur kapazitiven Auskopplung des Wechselspannungssignals stromauf der venösen Punktionskanüle (8) an einer zweiten Stelle der venösen Blutleitung (7) angeordnet sind.

12. Blutbehandlungsvorrichtung nach einem der Ansprüche 5 bis 8, **dadurch gekennzeichnet, dass** die venöse Blutleitung (7) stromauf der venösen Punktionskanüle (8) eine Schlauchverbindung (7c) zwischen einem ersten und einem zweiten Abschnitt aufweist, wobei die Mittel (26) zur kapazitiven Auskopplung des Wechselspannungssignals in dem ersten Abschnitt zwischen Punktionskanüle (8) und Schlauchverbindung (7c) angeordnet sind.

13. Blutbehandlungsvorrichtung nach einem der Ansprüche 5 bis 8, **dadurch gekennzeichnet, dass** die venöse Blutleitung (7) stromauf der venösen Punktionskanüle (8) eine Schlauchverbindung (7c) zwischen einem ersten und einem zweiten Abschnitt aufweist, wobei die Mittel (26) zur kapazitiven Auskopplung des Wechselspannungssignals in dem zweiten Abschnitt zwischen Blutkammer (3) und Schlauchverbindung (7c) angeordnet sind.

## Claims

1. Device for monitoring an access to a patient, in which a fluid is withdrawn from the patient via a first tubular conduit, which comprises a first patient connector, and the fluid is returned to the patient via a second tubular conduit, which comprises a second patient connector, in particular for monitoring a vascular access during an extracorporeal blood treatment in which a patient's blood is withdrawn from the patient via an arterial tubular conduit, which comprises an arterial patient connector with an arterial puncture cannula, and is returned to the patient via a venous tubular conduit, which comprises a venous patient connector with a venous puncture cannula,
in which the monitoring device comprises:
means (27a) which generate an AC voltage signal and which have a signal output (27b) with a first connector and second connector,
means (25) for capacitive injection of the AC voltage signal at a first location of one of the two tubular conduits,
means (26) for capacitive output of the AC voltage signal at a second location of the other of the two tubular conduits,
means (27c) which measure an AC voltage signal and which comprise a signal input (27d) with a first connector and second connector, in which the first connector of the signal input (27d) of the means (27c) for measuring an AC voltage signal is electrically connected to the means (26) for capacitive output of the AC voltage signal;
means (28) for producing an electrical connection between the first connector of the signal output (27b) of the means (27a) for generating an AC voltage signal and the means (25) for capacitive injection of the AC voltage signal, and for producing an electrical connection between the second connector of the signal output (27b) of the means (27a) for generating an AC voltage signal and ground;
means (II, 11, 11a) for producing an electrical connection between the fluid in the tubular conduits and ground, and
means (27e) for evaluating the measured AC voltage signal, which means (27e) interact with the means (27c) for measuring an AC voltage signal in such a way that a characteristic change of the amplitude of the measured AC voltage signal points to an incorrect vascular access;
**characterized in that**
the second connector of the signal input (27d) of the means (27c) for measuring an AC voltage signal is electrically connected to ground.

2. Device according to Claim 1, **characterized in that** the means (25) for capacitive injection of the AC voltage signal comprise a first body made of electrically conductive material for enclosing one of the two tubular conduits (6) at a first location.

3. Device according to Claim 1 or 2, **characterized in that** the means (26) for capacitive output of the AC voltage signal comprise a second body made of electrically conductive material for enclosing the other of the two tubular conduits (7) at a second location.

4. Device according to one of Claims 1 to 3, **characterized in that** the means (27e) for evaluating the measured AC voltage signal comprise means (27f) for comparing the measured AC voltage signal to a predetermined limit value, and, if the AC voltage signal is less than the limit value, this points to an incorrect vascular access.

5. Blood treatment device with a dialysis fluid circuit (II) which includes a dialysis fluid chamber (4) of a dialyzer (1) divided by a semipermeable membrane (2) into a blood chamber (3) and the dialysis fluid chamber (4), and a blood circuit (I) which includes the blood chamber (3) of the dialyzer (1), and in which an arterial tubular conduit (6) connected to an arterial puncture cannula (5) leads to the blood chamber of the dialyzer, while a venous tubular conduit (7) connected to a venous puncture cannula (8) issues from the blood chamber of the dialyzer, and with a device for monitoring the vascular access according to one of Claims 1 to 4.

6. Blood treatment device according to Claim 5, **characterized in that** the means for producing an electrical connection between the fluid in the tubular conduits and ground are arranged in the dialysis fluid circuit (I).

7. Blood treatment device according to Claim 6, **characterized in that** the means for producing an electrical connection between the fluid in the tubular conduits and ground are an electrical contact element (11a) which comes into contact with the dialysis fluid and is connected to ground potential.

8. Blood treatment device according to one of Claims 5 to 8, **characterized in that** the means (25) for capacitive injection of the AC voltage signal are arranged downstream of the arterial puncture cannula (5) at a first location of the arterial blood conduit (6).

9. Blood treatment device according to one of Claims 5 to 8, **characterized in that** the arterial blood conduit (6), downstream of the arterial puncture cannula (5), has a tube coupler (6c) between a first portion and a second portion, the means for capacitive injection (25) of the AC voltage signal being arranged in the first portion between puncture cannula (5) and tube coupler (6c).

10. Blood treatment device according to one of Claims 5 to 8, **characterized in that** the arterial blood conduit (6), downstream of the arterial puncture cannula (5), has a tube coupler (6c) between a first portion and a second portion, the means (25) for capacitive injection of the AC voltage signal being arranged in the second portion between tube coupler (6c) and blood chamber (3).

11. Blood treatment device according to one of Claims 5 to 8, **characterized in that** the means (26) for capacitive output of the AC voltage signal are arranged upstream of the venous puncture cannula (8) at a second location of the venous blood conduit (7).

12. Blood treatment device according to one of Claims 5 to 8, **characterized in that** the venous blood conduit (7), upstream of the venous puncture cannula (8), has a tube coupler (7c) between a first portion and a second portion, the means (26) for capacitive output of the AC voltage signal being arranged in the first portion between puncture cannula (8) and tube coupler (7c).

13. Blood treatment device according to one of Claims 5 to 8, **characterized in that** the venous blood conduit (7), upstream of the venous puncture cannula (8), has a tube coupler (7c) between a first portion and a second portion, the means (26) for capacitive output of the AC voltage signal being arranged in the second portion between blood chamber (3) and tube coupler (7c).

## Revendications

1. Dispositif de surveillance d'un accès à un patient, un liquide étant ponctionné sur le patient par l'intermédiaire d'une première ligne flexible, qui comporte un premier raccord pour patient, et le liquide étant réinjecté dans le patient par l'intermédiaire d'une deuxième ligne flexible qui comporte un deuxième raccord pour patient, en particulier pour la surveillance d'un abord vasculaire lors d'un traitement du sang extracorporel, au cours duquel le sang d'un patient est ponctionné sur le patient par l'intermédiaire d'une ligne artérielle, qui comporte un raccord artériel avec une canule de ponction artérielle, et est réinjecté dans le patient par l'intermédiaire d'une ligne veineuse, qui comporte un raccord veineux avec une canule de ponction veineuse, sachant que le dispositif de surveillance comporte :
■ des moyens (27a) pour générer un signal de tension alternative, qui comportent une sortie de signaux (27b) avec un premier et un deuxième raccord ;
■ des moyens (25) pour le couplage capacitif du signal de tension alternative au niveau d'un premier emplacement de l'une des deux lignes flexibles ;
■ des moyens (26) pour le découplage capacitif du signal de tension alternative au niveau d'un deuxième emplacement de l'autre des deux lignes flexibles ;
■ des moyens (27c) pour mesurer un signal de tension alternative, qui comportent une entrée de signaux (27d) avec un premier et un deuxième raccord, ledit premier raccord de l'entrée de signaux (27d) des moyens (27c) pour mesurer un signal de tension alternative étant relié électriquement aux moyens (26) pour le découplage capacitif du signal de tension alternative ;
■ des moyens (28) pour établir une liaison électrique entre le premier raccord de la sortie de signaux (27b) des moyens (27a) pour générer un signal de tension alternative et les moyens (25) pour le couplage capacitif du signal de tension alternative, et pour établir une liaison électrique entre le deuxième raccord de la sortie de signaux (27b) des moyens (27a) pour générer un signal de tension alternative et la masse ;
■ des moyens (II, 11, 11a) pour établir une liaison électrique entre le liquide dans les lignes flexibles et la masse ; et
■ des moyens (27e) pour analyser le signal de tension alternative mesuré, lesquels coopèrent avec les moyens (27c) pour mesurer le signal de tension alternative, de telle sorte que, lors d'une variation caractéristique de l'amplitude du signal de tension alternative mesuré, il en est déduit un abord vasculaire non correct ;
**caractérisé en ce que** le deuxième raccord de l'entrée de signaux (27d) des moyens (27c) pour mesurer un signal de tension alternative est relié électriquement à la masse.

2. Dispositif selon la revendication 1, **caractérisé en ce que** les moyens (25) pour le couplage capacitif du signal de tension alternative comportent un premier corps en matériau électroconducteur pour entourer l'une des deux lignes flexibles (6) au niveau d'un premier emplacement.

3. Dispositif selon la revendication 1 ou 2, **caractérisé en ce que** les moyens (26) pour le découplage capacitif du signal de tension alternative comportent un deuxième corps en matériau électroconducteur pour entourer l'autre des deux lignes flexibles (7) au niveau d'un deuxième emplacement.

4. Dispositif selon l'une quelconque des revendications 1 à 3, **caractérisé en ce que** les moyens (27e) pour analyser le signal de tension alternative mesuré comportent des moyens (27f) pour comparer le signal de tension alternative mesuré à une valeur limite prédéfinie, sachant que lorsque le signal de tension alternative est inférieur à ladite valeur limite, il en est déduit un abord vasculaire non correct.

5. Dispositif de traitement du sang, comportant un circuit de liquide de dialyse (II), qui contient une compartiment de liquide de dialyse (4) d'un dialyseur (1) séparé par une membrane (2) semi-perméable en un compartiment à sang (3) et un compartiment à liquide de dialyse (4), et un circuit sanguin (I), qui contient le compartiment à sang (3) du dialyseur (1), sachant qu'une ligne artérielle (6), qui est raccordée à une canule de ponction artérielle (5), mène vers le compartiment à sang du dialyseur, et une ligne veineuse (7), qui est raccordée à une canule de ponction veineuse (8), part du compartiment à sang du dialyseur, et comportant un dispositif de surveillance de l'abord vasculaire selon l'une quelconque des revendications 1 à 4.

6. Dispositif de traitement du sang selon la revendication 5, **caractérisé en ce que** les moyens pour établir une liaison électrique entre le liquide dans les lignes flexibles et la masse sont disposés dans le circuit du liquide de dialyse (I).

7. Dispositif de traitement du sang selon la revendication 6, **caractérisé en ce que** les moyens pour établir une liaison électrique entre le liquide dans les lignes flexibles et la masse sont un élément de contact (11a) électrique, qui entre en contact avec le liquide de dialyse et qui est mis au potentiel de la masse.

8. Dispositif de traitement du sang selon l'une quelconque des revendications 5 à 8, **caractérisé en ce que** les moyens (25) pour le couplage capacitif du signal de tension alternative sont disposés en aval de la canule de ponction artérielle (5) au niveau d'un premier emplacement de la ligne de sang artérielle (6).

9. Dispositif de traitement du sang selon l'une quelconque des revendications 5 à 8, **caractérisé en ce que** la ligne de sang artérielle (6) comporte, en aval de la canule de ponction artérielle (5), une liaison par flexible (6c) entre un premier et un deuxième tronçon, sachant que les moyens (25) pour le couplage capacitif du signal de tension alternative dans le premier tronçon sont disposés entre la canule de ponction (5) et la liaison par flexible (6c).

10. Dispositif de traitement du sang selon l'une quelconque des revendications 5 à 8, **caractérisé en ce que** la ligne de sang artérielle (6) comporte, en aval de la canule de ponction artérielle (5), une liaison par flexible (6c) entre un premier et un deuxième tronçon, sachant que les moyens (25) pour le couplage capacitif du signal de tension alternative dans le deuxième tronçon sont disposés entre la liaison par flexible (6c) et le compartiment à sang (3).

11. Dispositif de traitement du sang selon l'une quelconque des revendications 5 à 8, **caractérisé en ce que** les moyens (26) pour le découplage capacitif du signal de tension alternative sont disposés en amont de la canule de ponction veineuse (8) au niveau d'un deuxième emplacement de la ligne de sang veineuse (7).

12. Dispositif de traitement du sang selon l'une quelconque des revendications 5 à 8, **caractérisé en ce que** la ligne de sang veineuse (7) comporte, en amont de la canule de ponction veineuse (8), une liaison par flexible (7c) entre un premier et un deuxième tronçon, sachant que les moyens (26) pour le découplage capacitif du signal de tension alternative dans le premier tronçon sont disposés entre la canule de ponction (8) et la liaison par flexible (7c).

13. Dispositif de traitement du sang selon l'une quelconque des revendications 5 à 8, **caractérisé en ce que** la ligne de sang veineuse (7) comporte, en amont de la canule de ponction veineuse (8), une liaison par flexible (7c) entre un premier et un deuxième tronçon, sachant que les moyens (26) pour le découplage capacitif du signal de tension alternative dans le deuxième tronçon sont disposés entre le compartiment à sang (3) et la liaison par flexible (7c).
